# EUROPÄISCHE PATENTSCHRIFT

(11) **EP 0 767 949 B1**
(45) Veröffentlichungstag und Bekanntmachung des Hinweises auf die Patenterteilung: **02.02.2000**
(21) Anmeldenummer: 95921769.6
(22) Anmeldetag: 29.05.1995
(51) Int. Cl.: G10K 11/22, G10K 11/08

(54) **VERFAHREN UND VORRICHTUNG ZUR HERSTELLUNG VON KUGELWELLEN IM ULTRASCHALLBEREICH**
PROCESS AND DEVICE FOR GENERATING SPHERICAL WAVES IN THE ULTRASOUND RANGE
PROCEDE ET DISPOSITIF PERMETTANT DE PRODUIRE DES ONDES SPHERIQUES DANS LA GAMME ULTRASONORE

(30) Priorität: 30.05.1994 DE 4418830; 06.12.1994 US 349879
(43) Veröffentlichungstag der Anmeldung: 16.04.1997
(73) Patentinhaber: Sonident Anstalt Liechtensteinischen Rechts, 9490 Vaduz (LI)
(72) Erfinder: BICZ, Wieslaw, PL-52-011 Wroclaw (PL)
(74) Vertreter: Funck-Hartherz, Anna-Eleonore, Dipl.-Phys.
(86) Internationale Anmeldenummer: EP9502031
(87) Internationale Veröffentlichungsnummer: WO9533258

(56) Entgegenhaltungen:
- DE-C- 410 523

## Beschreibung

Die Erfindung bezieht sich auf ein Verfahren zur Herstellung von perfekten Kugelwellen im Ultraschallbereich sowie auf eine Vorrichtung zur Erzeugung dieser Kugelwellen.

Um eine möglichst gute Kugelwelle im Ultraschallbereich herstellen zu können, muß man nach dem bisherigen Stand der Technik einen sehr kleinen Sender, möglichst einen Punktsender verwenden, der nur eine geringe Schallintensität besitzt. Die Erfahrung zeigt jedoch, daß auch bei kleinen Sendern keine phasenrichtigen Wellen gesendet werden. Unter einer phasenrichtigen Welle versteht man eine Welle, deren erste Wellenfront eine perfekte Kugelform hat und auch die nachfolgenden Wellenfronten dazu parallel verlaufen.

Eine perfekte Kugelwelle ist bei allen Ultraschallgeräten dann von Bedeutung, wenn die Phase der Welle berücksichtigt werden muß. Alle Ultraschallgeräte wären bei Verwendung von perfekten Kugelwellen wesentlich verbessert, wenn es auch bei einigen der Geräte nicht von so großer Bedeutung ist.

Die der Erfindung zugrundeliegende Aufgabe besteht darin, ein Verfahren und eine Vorrichtung zur Erzeugung von Ultraschallwellen vorzuschlagen, deren erste Wellenfront eine perfekte Kugelform aufweist und ebenso die nachfolgenden Wellenfronten. Die bevorzugte Ultraschallfrequenz liegt im Bereich von 5 - 15 MHz.

Eine weitere Aufgabe der Erfindung besteht darin, daß die Intensität des Schalls mit der Entfernung von der Symmetrieachse gleichmäßig abfällt, also am besten eine Gauß' sehe Verteilung aufweist, die mit den bekannten klassischen Verfahren und Vorrichtungen kaum zu erreichen ist. Eine Gauß'sche Verteilung wird deshalb bevorzugt, weil dabei die Intensität in der Mitte der Welle am größten ist und an den Seiten stark abfällt, denn eine zu große Intensität im Randbereich kann zu unerwünschten Störungen führen.

Zur Lösung der gestellten Aufgabe, nämlich der Erzeugung einer perfekten Kugelwelle mit einer Gauß'schen Intensitätsverteilung, wird ein Verfahren vorgeschlagen, das eine Verdichtung der von einem Ultraschallsender ausgehenden Ultraschallwelle durch einen Wellenleiter bis zu einer einmodalen Wellenleitung vorsieht, wobei am Ende des Wellenleiters die Ultraschallwelle als perfekte Kugelwelle in den freien Raum abgegeben wird. Die ausgesendete Welle wird also erfindungsgemäß durch sie umgebende Einflüsse in Form eines Wellenleiters einer Beeinflussung unterworfen. Die Verdichtung der von dem Ultraschallwandler ausgehenden Welle erfolgt dabei langsam und kontinuierlich.

Eine Vorrichtung zur Durchführung dieses Verfahrens ist aus Anspruch 3 zu entnehmen. Sie ist folgendermaßen aufgebaut. Es wird ein ganz normaler Ultraschallwandler mit einem relativ großen Durchmesser z. B. von einigen Millimetern verwendet. Die Welle, die ein solcher Wandler aussendet, ist nicht homogen, d. h. sowohl ihre räumliche wie auch ihre zeitliche Verteilung (Amplitude und Phase) sind von einer ebenen Welle oder Kugelwelle weit entfernt. An diesen Ultrallschallwandler ist gemäß der Erfindung in Senderichtung ein Trichter angeschlossen, der an seinem Ende in ein Rohr mündet. Der Durchmesser des Rohres ist dabei größer als die Wellenlänge der Ultraschallwelle. Es hat sich gezeigt, daß die besten Ergebnisse erzielt werden, wenn der Durchmesser des Rohres cirka zehnmal größer ist als die Wellenlänge der Ultraschallwelle. Der Trichter ist dabei am besten so gestaltet, daß die gesamte Wellenenergie in das Rohr geführt wird, das dann wie ein einmodaler Wellenleiter wirkt, und die Welle, die den Wellenleiter verläßt, verhält sich dann so, als ob sie von einem Punkt gesendet würde. Sie ist perfekt phasenrichtig, d. h. sie ist eine perfekte Kugelwelle und besitzt außerdem die gewünschte Gauß'sche Intensitätsverteilung. Eine perfekte Kugelwelle ist eine solche Ultraschallwelle, in der die Phase jeder Wellenfront die Fläche einer Kugel hat. Infolge der durch die Erfindung gegebenen Möglichkeit, einen großen Sender zu verwenden, wird die zur Verfügung stehende Energie der Schallwelle um einen wesentlichen Betrag gegenüber einer normalen Punktquelle erhöht.

Der Trichter und das Rohr gemäß einer erfindungsgemäßen Ausgestaltung bestehen vorzugsweise aus einem Festkörper. Es hat sich herausgestellt, daß ein schallabsorbierendes Material, vorzugsweise Kunststoff, sich besonders eignet.

Der Trichter und das Rohr können in einem Flüssigkeitsbad oder in einer Gasatmosphäre angeordnet sein. Auch ist es möglich, den Trichter und das Rohr außen von einem Festkörper umgeben zu lassen wobei auch innen ein fester Körper vorgesehen ist, der aber für die Ultraschallwellen durchlässig sein muß. Die Länge des als Wellenleiter dienenden Rohres bemißt sich nach einer solchen Führung der Ultraschallwelle, daß an dem Ende des Rohres, wie schon erwähnt, perfekte Kugelwellen austreten. Das Rohr kann beliebig gebogen sowie geführt werden, so daß der Startpunkt der Kugelwelle wunschgemäß placiert werden kann.

Es besteht auch die Möglichkeit, anstelle des an den Trichter sich anschließenden Rohres die verdichtete Welle einem anderen Wellenleiter, wie z. B. einem Glasfaserdraht am Ende des Trichters zuzuleiten.

Es hat sich gezeigt, daß der Ultraschallwandler gemäß Erfindung in einem ziemlich großen Frequenzspektrum, z. B. von 2 bis 20 MHz, perfekte Kugelwellen hervorbringt.

Durch die Erfindung werden somit inhomogene Wellen in perfekte Kugelwellen umgewandelt.

Die vorgenannten Aufgaben, Merkmale und Vorteile werden noch weiter durch die folgende Beschreibung der beigefügten Zeichnung erläutert. Dabei zeigen:
- Fig. 1: eine Schnittansieht der Vorrichtung gemäß Erfindung in schematischer Darstellung,
- Fig. 2: die Gauß'sche Intensitätsverteilung von den gemäß Erfindung erzeugten Ulstraschallwellen,
- Fig. 3: eine mögliche Anwendung des Kugelwellensenders gemäß der Erfindung.

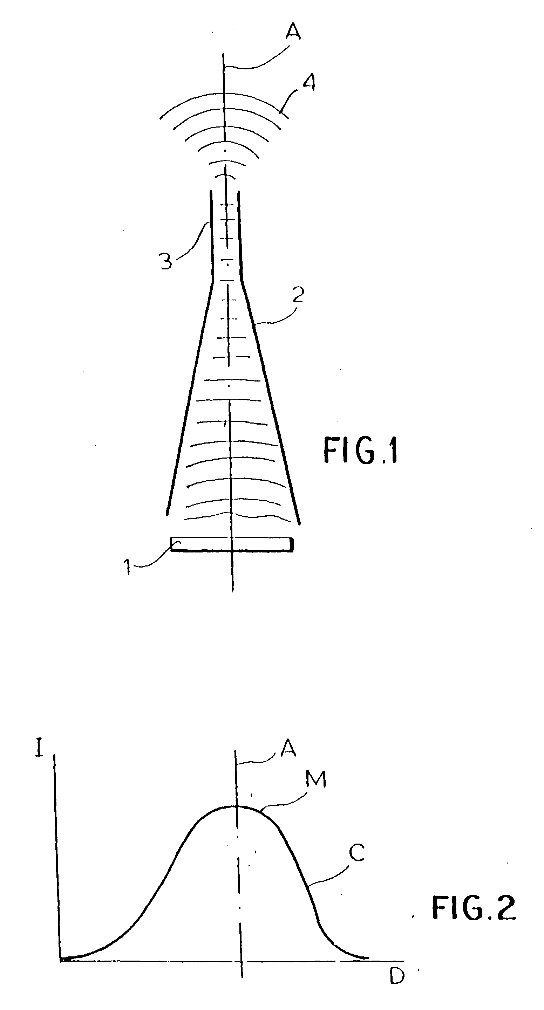

Wie aus Fig. 1 ersichtlich, ist ein Ultraschallsender 1, der wesentlich größer als eine Punktquelle gestaltet ist, die zur Erzeugung von Kugelwellen normalerweise als notwendig erachtet wird, an der breiten Seite des Trichters 2 angeordnet, der die Engergie und die Schallwellen bündelt und verdichtet, die sich durch den Trichter fortpflanzen. An das Auslassende des Trichters schließt sich ein Rohr 3 an und bildet einen ein modalen Wellenleiter aus welchem die Ultraschallwellen 4 phasenrichtig und mit kugeliger Wellenfront austreten.

Die Wellen bilden ein Feld, dessen Intensität an jeder Seite der Symmetrieachse A entsprechend einer Gauß'schen Kurve C mit einem Maximum M entlang der Symmetrieachse gemäß dem Diagramm abfallen, in welchem die Intensität I in Abhängigkeit von der Entfernung D, wie in Fig. 2 gezeigt, aufgezeichnet ist.

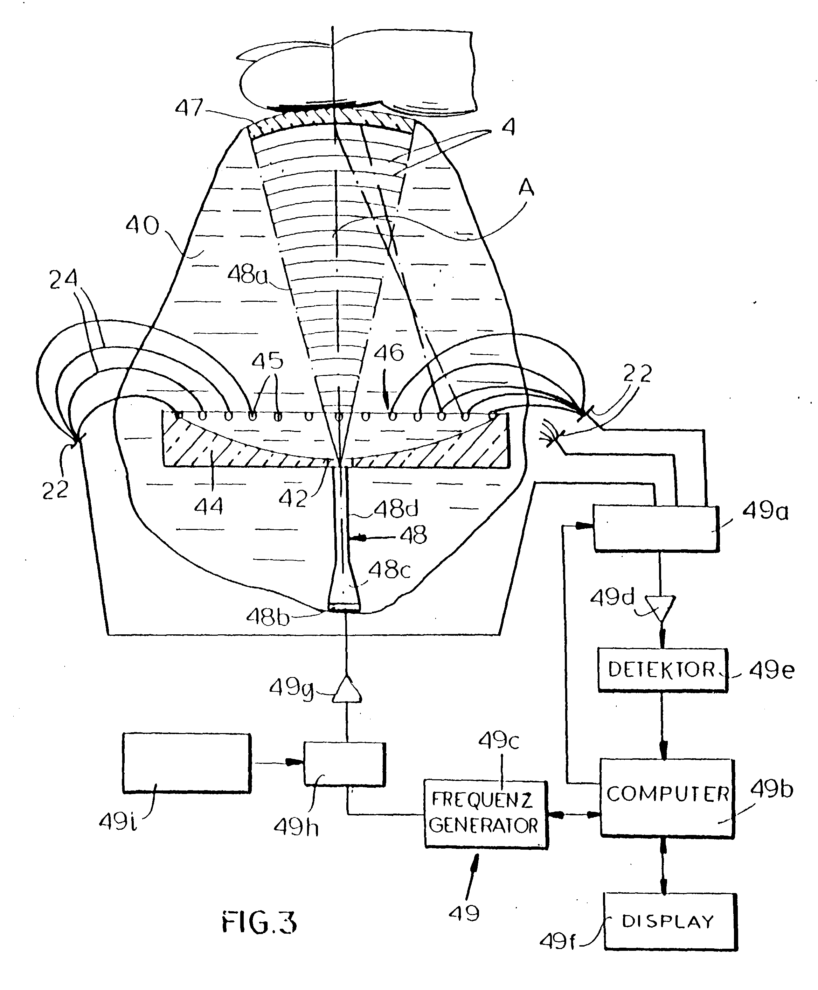

Der Sender von Ultraschallwellen in Kugelform gemäß Fig. 1 kann in einer Vorrichtung gemäß Fig. 3 verwendet werden, z.B. zur Ermittlung von Fingerabdrücken von Personen.

Die Vorrichtung gemäß Fig. 3 besteht aus einem Ultraschallgenerator 48, gebildet von dem Sender 48b, dessen Wellen in dem Trichter 48c verdichtet werden und der mit dem Wellenleiterrohr 48d kommuniziert, so daß an einer öffnung 42 des Trägers 44 die Ultraschallwellen in ein flüssiges Medium z. B. Wasser eintreten und sich darin in einer solchen Weise fortpflanzen, daß ein Wellenbündel 48a strahlenförmig auf eine Platte 47 fällt. Die gesamte Fläche der Platte 47 wird von den Kugelwellen bestrahlt.

Die Flüssigkeit bildet das schallübertragende Medium zwischen der Ultraschallquelle 48 und der Platte 47. Der Träger 44 hat eine sphärische Oberfläche 43. Die Platte 47, die durchlässig für Ultraschallwellen ist, kann aus Glas hergestellt sein und ist eine konvex-konkave von konstanter Dicke, wobei die konvexe Seite als Auflagefläche für das zu betrachtende Objekt, z. B. die Fingerkuppe dient.

Auf der Oberfläche des Trägers 44 sind anstelle von einzelnen Wandlern, die auch in dieser Ausführung Verwendung finden können, zahlreiche kleine Empfänger 45 vorgesehen und zwar dicht beieinander in einem Empfängerkollektor oder in einem ringförmigen Feld 46. Die Oberfläche ist vorzugsweise sphärisch.

Die Empfängerkollektoren 45 können über Wellenleitern mit Wandlern 22 verbunden sein, die der Reihe nach mit einem (Abtaster) Scanner 49a des Schaltkreises 49 verbunden sind.

Die einzelnen Wandler 22 werden nacheinander durch den Scanner 49a unter der Kontrolle des Computers 49b abgetastet, welcher mit dem Frequenzgenerator verbunden sein kann, so daß er mit jedem Frequenzwechsel des Empfangswandlers in Folge abgetastet werden kann.

Die abgetastete Leistung kann bei 49d verstärkt werden und wird dann dem Detektor 49e zugeführt, mit dessen Signal der Computer 49b gefüttert wird und der es an ein Display 49f weitergibt, das das Muster des Fingerabdruckes oder andere Eigenschaften abbildet.

Der Frequenzgenerator 49c kann einen Verstärker 49d über ein Tor 49h speisen, der den Sender 48b füttert. Das Tor 49h wird von einem Impulsgenerator oder einer anderen Impulsquelle 49i gesteuert.

Die vorbeschriebene Vorrichtung bestimmt die Konturen eines sich auf der Platte 47 befindlichen Objektes.

## Patentansprüche

1. Verfahren zur Herstellung von Kugelwellen im Ultrschallbereich,
**dadurch gekennzeichnet,**
daß eine Verdichtung der von einem Ultraschallsender ausgehenden Ultraschallwelle durch einen Wellenleiter bis zu einer einmodalen Wellenleitung erfolgt, an dessen Ende die Ultraschallwelle als Kugelwelle in den freien Raum abgegeben wird.

2. Verfahren nach Anspruch 1,
**dadurch gekennzeichnet**,
daß die Verdichtung langsam und kontinuierlich erfolgt.

3. Vorrichtung zur Durchführung des Verfahrens nach Anspruch 1 oder 2,
**dadurch gekennzeichnet,**
daß nächst dem Ultraschallsender (1) in Senderichtung ein Trichter (2) angeordnet ist, der an seinem Ende in ein Rohr (3) einmündet.

4. Vorrichtung nach Anspruch 3,
**dadurch gekennzeichnet,**
daß das Rohr (3) einen Durchmesser aufweist, der größer ist als die Wellenlänge der gesendeten Ultraschallwelle.

5. Vorrichtung nach Anspruch 4,
**dadurch gekennzeichnet,**
daß das Rohr (3) einen Durchmesser in der Größenordnung von 10 Wellenlängen der gesendeten Ultraschallwelle aufweist.

6. Vorrichtung nach Anspruch 3,
**dadurch gekennzeichnet,**
daß anstelle des Rohres (3) ein anderer Wellenleiter zur Führung der Ultraschallwelle angeordnet ist.

7. Vorrichtung nach Anspruch 3,
**dadurch gekennzeichnet,**
daß der Trichter (2) und das Rohr (3) aus einem Festkörper besteht.

8. Vorrichtung nach Anspruch 7,
**dadurch gekennzeichnet,**
daß der Trichter (2) und das Rohr (3) aus einem schallabsorbierenden Material, vorzugweise Kunststoff, besteht.

9. Vorrichtung nach Anspruch 3,
**dadurch gekennzeichnet,**
daß der Trichter (2) und das Rohr (3) in einem Flüssigkeitsbad oder einer Gasatmosphäre angeordnet ist.

10. Vorrichtung nach Anspruch 3,
**dadurch gekennzeichnet,**
daß der Trichter (2) und das Rohr (3) innen und außen aus einem Festkörper bestehen.

11. Vorrichtung nach einem oder mehreren der vorhergehenden Ansprüche,
**dadurch gekennzeichnet,**
daß das Rohr (3) gerade oder gebogen verläuft.

## Claims

1. Process to produce spherical ultrasonic waves
characterized in that
a compression of the ultrasonic wave coming from the ultrasound transmitter is effected through a wave conductor to a single modal wave conducting and on its end the ultrasonic wave is emitted as spherical wave in space.

2. Process according patent claim 1
characterized in that
the compression is effected slowly and continually.

3. Device to realize the process according patent claim 1 or 2
characterized in that
nearest to the ultrasound transmitter (1) in transmitting direction a funnel (2) is placed leading on its end in a pipe (3).

4. Device according patent claim 3
characterized in that
the diameter of the pipe (3) is bigger than the wave-length of the transmitted ultrasonic wave.

5. Device according patent claim 4
characterized in that
the diameter of the pipe (3) in corresponding approximately with ten times of the wave-length of the transmitted ultrasonic wave.

6. Device according patent claim 3
characterized in that
in place of the pipe (3) another wave conducting is used to conduct the ultrasonic wave.

7. Device according patent claim 3
characterized in that
the funnel (2) and the pipe (3) consist of an solid substance.

8. Device according patent claim 7
characterized in that
the funnel (2) and the pipe (3) consist of a sound absorbent material preferably synthetic substance.

9. Device according patent claim 3
characterized in that
the funnel (2) and the pipe (3) are placed in a bath of a liquid or in an atmosphere of gas.

10. Device according patent claim 3
characterized in that
the funnel (2) and the pipe (3) consist on the inside and on the outside of a solid substance.

11. Device according one or several of the previous patent claims
characterized in that
the pipe (3) is running straight or bended.

## Revendications

1. Procédé à la production d'ondes ultrasonores sphériques,
caracterise par le fait,
qu'une compression d'une onde ultrasonore émise par un émetteur ultrasonique est effectuée par un conducteur d'ondes jusqu'à une conduite modale unique et au bout duquel l'onde ultrasonore est émise en qualité d'onde sphérique dans l'espace.

2. Procédé après revendication 1,
caracterisé par le fait,
que la compression est effectuée lentement et continuellement.

3. Dispositif pourla réalisation du procédé après revendication 1 ou 2,
caracterisé par le fait,
que tout près de l'émetteur ultrasonique (1) dans la direction de l'émission un cornet (2) est arrangé qui débouche à son bout dans un tuyau (3).

4. Dispositif après revendication 3,
caracterisé par le fait,
que le diamètre du tuyau (3) est plus grand que la longueur de l'onde ultrasonore émise.

5. Dispositif après revendication 4,
caracterisé par le fait,
que le diamètre du tuyau (3) correspond approximativement à dix fois de la longueur de l'onde ultrasonore émise.

6. Dispositif apres revendication 3,
caracterisé par le fait,
qu'au lieu du tuyau (3) un autre conducteur d'ondes est arrangé pour la conduction de l'onde ultrasonore.

7. Dispositif apres revendication 3,
caractérisé par le fait,
que le cornet (2) et le tuyau (3) consistent d'un corps solide.

8. Dispositif après revendication 7,
caracterisé par le fait,
que le cornet (2) et le tuyau (3) consistent d'un matériel absorbent le son, préferablement en matière plastique.

9. Dispositif après revendication 3,
caracterisé par le fait,
que le cornet (2) et le tuyau (3) sont arrangé dans un bain d'un liquide ou dans une atmosphère de gaz.

10. Dispositif après revendication 3,
caractérisé par le fait,
que le cornet (2) et le tuyau (3) consistent à l'interieur et à l'exterieur d'un corps solide.

11. Dispositif après une ou plusieurs des revendications mentionnes ci-dessus,
caracterisé par le fait,
que le tuyau (3) est arrangé tout droit ou en courbure.
